Europäisches Patentamt

European Patent Office

Office européen des brevets

(11) Publication number: **0 025 309**
A1

(12) **EUROPEAN PATENT APPLICATION**

(21) Application number: **80302949.5**

(22) Date of filing: **26.08.80**

(51) Int. Cl.³: **C 02 F 3/12**
**C 12 M 1/00, B 01 J 8/20**

(30) Priority: **23.08.79 US 68765**

(43) Date of publication of application:
**18.03.81 Bulletin 81/11**

(84) Designated Contracting States:
**AT BE CH DE FR GB IT LI NL SE**

(71) Applicant: **ECOLOTROL INC.**
**1211 Stewart Avenue**
**Bethpage, New York 11714(US)**

(72) Inventor: **Hickey, Robert F.**
**284 Locust Street**
**Florence Massachusetts 01060(US)**

(74) Representative: **Warden, John C. et al,**
**R.G.C. JENKINS & CO. 53/64 Chancery Lane**
**London WC2A 1QU(GB)**

(54) Downflow bioreactor.

(57) A biological treatment technique in which waste water or other liquid to be processed is conducted downwardly through a bed of carrier particles to which biological material is attached. In a system for carrying out this technique, the bed (13) is suspended within a vertical reactor (10) by the liquid, the carrier particles having a specific gravity that is less than that of the liquid. The liquid is introduced into the upper end of the bed (12) to pass through the particles and exits through the bottom of the reactor (21). Excess biological material found on the particles is removed therefrom (16L).

Fig.1.

EP 0 025 309 A1

## BACKGROUND OF INVENTION

Field of Invention:

This invention relates generally to biological treatment techniques, and more particularly to a reactor in which waste water or other liquid to be processed is conducted downwardly through a bed of small carrier particles to which biological material is attached.

Prior Art:

Biologically-catalyzed reactions which occur in nature and make use of bacteria, yeast, mold, enzymes and algae, are exploited in industrial processing, as in the fermentation of alcoholic beverages, in the production of drugs and in the treatment of sewage or waste water.

While the invention will be described as it applies to waste water treatment, it is to be understood that reactor systems in accordance with the invention have other practical applications.

Systems involving biologically-catalyzed reactions may be broadly divided into two main categories, the first being the suspended growth system and the second the fixed film reactor.

In a suspended growth system, micro-organisms are suspended in the liquid medium to be treated in a reaction vessel or tank, the suspension being effected by stirring the liquid by mechanical agitators or by gas injection. When the system is designed to operate in a continuous manner, the usual practice is to provide a second tank whose function is to settle out organisms suspended in the treated effluent drawn from the reaction vessel and to return these organisms to the vessel for re-use. Though a suspended growth system effects the desired treatment, it entails the use of large tanks and dictates prolonged treatment periods. This system, therefore, has distinct economic drawbacks.

In a fixed film reactor system, the biological catalysts are not in suspension but are attached to an inert support surface to react with the liquid. An early form of this system is the trickling filter in which liquid to be treated is trickled over a bed of stone to which the micro-organisms are attached. Other forms include submerged filters in which the liquid to be processed is passed through a packed bed of media in either the upward or downward direction. The biological efficiency of this process is directly related to the amount of surface afforded by the packed media for biological attachment.

In order to augment the available square feet of surface for microbial attachment per cubic foot of reactor volume, in recent years various forms of synthetic media have been developed for this purpose. But this development has not overcome many of the significant operating difficulties of the packed bed system, and in fact has aggravated some of these difficulties.

In a system operated in a packed state, there is no direct control over the amount of biological growth contained therein, and the system can eventually become clogged with such growth. As a consequence, channeling occurs in the reactor and the liquid to be treated bypasses the clogged regions and thereby makes no contact with the micro-organisms therein.

Clogging can be checked by periodically backwashing the packed bed system; but to do so requires that operation of the system be discontinued, thereby disrupting the treatment process. In the treatment of waste water, a packed bed system has the advantage of filtering out suspended material from the waste water while biological treatment is in progress. But this advantage is also a drawback, for it gives rise to plugging and channeling problems.

In the field of fixed film reactors, the most recent development is the use of fluidized-beds to carry out biologically-catalyzed reactions. In existing forms of fluidized bed reactors, the liquid to be treated is passed upwardly through a bed of small carrier particles whose specific gravity is greater than that of the liquid, so that the particles have a tendency to sink. However, the upward flow rate of the liquid through the bed is sufficient to fluidize the particles.

0025309

In the fluidized-bed reactor, the small carrier particles afford a vast, continuously moving surface area for microbial attachment, and at the same time obviate plugging problems associated with packed bed reactors. While the introduction of fluidized bed technology represents a significant advance in the art of biologically-catalyzed reactions, this technique, as presently practiced, suffers from certain drawbacks, the most serious of which lies in its high energy requirements.

A considerable amount of energy must be expended in producing the upward flow of liquid serving to fluidize the particles in the bed. In order to somewhat reduce energy consumption, it is the present practice to make use of carrier particles of such size and density that it becomes possible to effect fluidization at relatively low flow velocities. However, if a gaseous reactant is present or if gas is generated as a by-product of the reaction, the resultant gas bubbles may attach themselves to the carrier particles, thereby causing the particles to float upwardly and out of the reactor.

Another problem encountered in upflow fluid bed reactors is the difficulty of achieving an even flow distribution with minimal turbulence and wear on the flow distributor. The reason for this problem is that with an upflow technique, the particles must of necessity be in contact with the distributor therebelow, as a consequence of which flow instabilities such as channeling and packing are difficult to avoid. Moreover, wear of the distributor by the abrading particles may become excessive. In order, therefore, to attain approximately even distribution in an upflow fluidized bed reactor, valuable energy is sacrificed in the distributor arrangement.

Prior Art References:

The following references are generally pertinent to the discussion of the prior art:

A. Fluidized Beds

1. Scott et al., patent no. 4,032,407, June 28, 1977, "Tapered Bed Bioreactor." This patent discloses an up-flow fluidized bed utilizing media whose specific gravity is heavier than that of the liquid medium. The feature of interest is a tapered reactor which allows the system to more easily accommodate varying flows than a straight walled reactor.

2. Jeris et al, patent no. 3,846,289, November 5, 1974, "Waste Treatment Process"; and patent no. 3,956,129, May 11, 1976, "Waste Water Apparatus." An up-flow fluidized bed is disclosed utilizing media whose specific gravity is heavier than that of the liquid medium. The process and apparatus are directed toward denitrification of wastewater.

3. Jeris, patent no. 4,009,098, February 22, 1977, "Waste Treatment Process," and patent no. 4,009,105, February 23, 1977, "Waste Treatment Apparatus." An up-flow fluidized bed is applied to aerobic BOD removal of wastewater.

4. Jeris, patent no. 4,009,105, February 23, 1977, "Waste Treatment Apparatus." An up-flow fluidized bed applied to denitrification of wastewater.

5. Benzaria, patent no. 4,089,162, April 25, 1978, "Method of Adsorption by Activated Charcoal in a Lower Fluidized Bed and Upper Fixed Bed." A conventional up-flow fluidized bed for adsorption, not biochemical reactions.

B.  Expanded Beds

1.  Weber et al., "Expanded-Bed Adsorption for Treatment of Sewage Effluents," Water - 1970, No. 107, Vol. 67, p. 541. This reference deals with an up-flow expanded bed process for the physical adsorption or organic pollutants onto activated carbon.

2.  Johnson, R.L., and Baumann, E.R., "Advanced Organics Removal by Pulsed Adsorption Beds," JWPCF, 43, No. 8, 1640 (1971).  This reference deals with a "quasi" expanded bed for adsorption in an up-flow operation using media whose specific gravity is heavier than the liquid medium.

C.  Packed Beds

1.  Balakrishnan, et al., "Organics Removal by Selected Trickling Filter Media, " Water & Wastes Engineering, January 1969.  This relates to operation of a conventional trickling filter using an improved support media.  The liquid to be treated is trickled over the packing media which is placed in the reactor.  The flow is in a downward direction but the media is not submerged in the liquid.

2.  Haug, R.T., and McCarty, P.L., "Nitrification with Submerged Filters, " JWPCF, 44, No. 11, p. 2086 (1972).  The flow is passed upwardly through a column of 1 inch·diameter stone media submerged in the liquid in a packed state.

3.  English et al., "Denitrification in Granular Carbon and Sand Columns," JWPCF, 46, No. 1, p. 28 (1974). Wastewater is passed downwardly through a bed of sand or activated carbon in a packed state.

4. Francis et al., patent no. 4,043,936, August 23, 1977, "Biological Denitrification of High Concentration Nitrate Waste." This deals with an up-flow packed bed for denitrification.

5. F.O. Mixon, "Moving Bed Filtration of Municipal Waste," JWPCF 45, No. 8, p. 1718 (1973). This discloses a process in which a liquid is passed in an upward direction through the media and is directed to physical filtration, not biological treatment. The media is in a packed state and is continuously removed and cleaned without interrupting the process, but the cleaning operation is for removing suspended solids entrained by the particles and not biological growth.

## SUMMARY OF INVENTION

In view of the foregoing, the main object of this invention is to provide a technique and a system based thereon for carrying out biologically-catalyzed reactions in which waste water or other liquid to be processed is conducted downwardly through a bed of carrier particles.

A significant feature of a technique in accordance with the invention is that it retains the conventional high rate advantages characteristic of fluidized bed reactors while obviating many of the drawbacks incident to such reactors, such as flow distribution and bubble attachment problems.

Yet another object of this invention is to provide a technique of the above type which carries out biologically-catalyzed reactions while realizing some degree of mechanical filtration if desired, and the liquid is so amenable without the need to interrupt the process for backwashing.

It is also an object of the invention to provide a system of the above-noted type which operates reliably and efficiently, and which can be manufactured and installed at relatively low cost.

Briefly stated, these and other objects of the invention are attained in a technique and system based thereon in which the liquid to be treated is conducted downwardly in a vertically disposed reactor through a bed of packed, expanded or fluidized carrier particles to which biota is attached whose specific gravity is less than that of the liquid, as a consequence of which the bed is suspended in the reactor.

The particles in the bed to which the biota is attached are enlarged in the course of operation by the growth of the biota, excess material being removed therefrom. The treated liquid is withdrawn from the bottom of the reactor.

OUTLINE OF DRAWINGS

For a better understanding of the invention as well as other objects and further features thereof, reference is made to the following detailed description to be read in conjunction with the accompanying drawings, wherein:

Fig. 1 illustrates a generalyzed system which operates in accordance with the invention;

Fig. 2 illustrates the operation of the system in the fluidized-bed mode;

Fig. 3 illustrates the operation of the system in the expanded or packed-bed mode; and

Fig. 4 illustrates the operation of the system in a combination of the fluidized-bed and expanded or packed-bed modes.

DESCRIPTION OF INVENTION

Basic Principles:

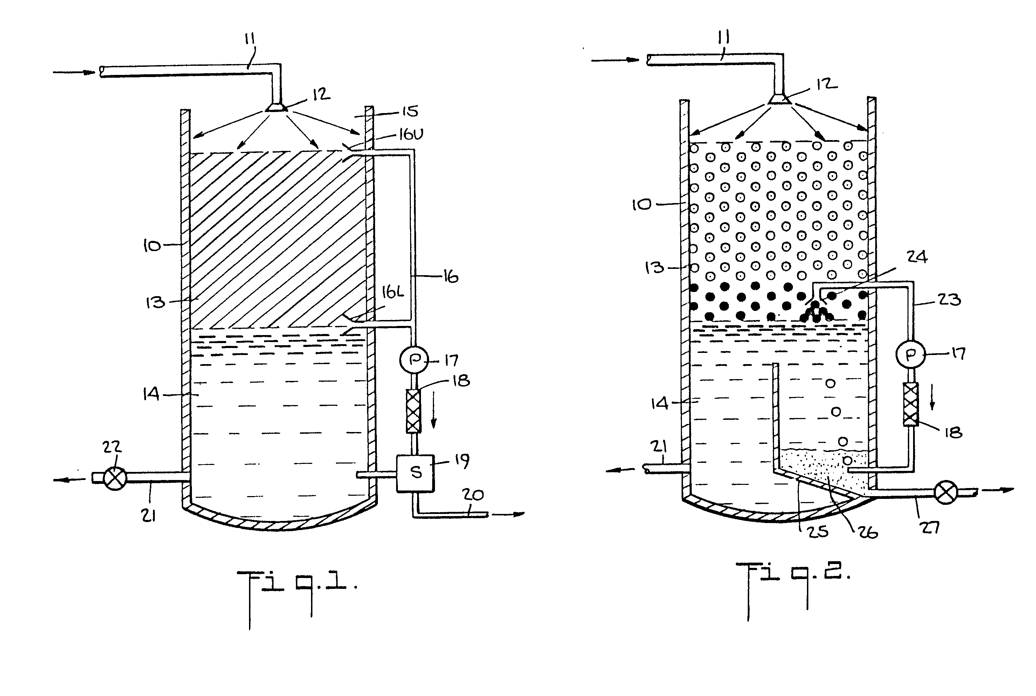

Referring now to Fig. 1, there is schematically shown a biologically-catalyzed reaction system illustrative of the basic concepts underlying the invention.

The waste water or liquid to be treated is introduced into a vertical reactor 10 through an inlet pipe 11. In practice, the liquid may be pumped into inlet pipe 11, or gravity flow may be used because of the minimal headlosses in this system. Pipe 11 leads into a distributor 12 from which the liquid enters, is sprayed or dispersed into the reactor.

0025309

Reactor 10 is constructed in any suitable vertical configuration of a material appropriate to the liquid being treated and it may have a circular, square, rectangular, conical or other cross-sectional geometry, depending on specific design objectives. In practice, the reactor may be sealed to permit pressurization thereof to maintain oxygen-free conditions or to collect gaseous reaction products.

Disposed in reactor 10 is a suspended bed 13 of particles to which biota is attached having a specific gravity which is less than that of the liquid being treated. Suspended bed 13 is interposed in the reactor between an underlying pool or bath 14 of the treated liquid which has passed through, and a gas head 15 thereabove. In the case of an unsealed reactor, the gas head may be the atmosphere. Because the particles of the bed are effectively trapped in the boundary between two fluids (gas head 15 and the liquid being treated), the liquid distributor 12 may be a simple weir arrangement. Although significant flow distribution advantages may be gained in the system because incoming liquid is caused to flow downwardly through a gas space above the particles rather than upwardly by liquid injection into the particles as in a conventional fluidized-bed reactor, it is to be understood that in practice distributor 12 may be submerged in the media and still take advantage of other benefits of the system.

The size and density of the particles which constitute bed 13 are a matter of design choice, depending on the treatment objectives, but in no event can the particles with the attached biota have a specific gravity exceeding that of the liquid being treated; for it is essential that the particles remain suspended above liquid bath 14. Preferably, the particles in the media are in a size range of 0.1 mm to 2.0 cm. In most liquid treatment applications, the particles will have a specific gravity that is less than 1.0. The media for this purpose can be constituted by cork, wood or plastic particles, hollow glass beads or other light-weight material.

In practice, the media can be seeded with any biological catalyst in the manner described in the above-identified Scott patent. Suitable catalysts include bacteria, yeast, algae, mold or immobilized enzymes. The type of organism seeded to the media is, of course, determined by the treatment objective.

As biological growth accumulates on the media, a point is reached where it becomes necessary to remove excess growth from the particles. If growth is unchecked, the bed particles with attached biota become enlarged to a degree resulting in either agglomeration, plugging or carry out, thereby reducing the biological surface area per unit volume and impairing the efficiency of the reactor. Excess growth can be removed by means of a rotating blade or other abrasion devices operating within the reactor.

The preferred technique for removing excess growth is by means external to the reactor, as shown in Fig. 1, wherein the particles from the bed are drawn from the upper and/or lower end of bed 13 depending on the mode of operation of the bed through reactor ports 16U and 16L coupled to a line 16 by a pump 17. The pumped particles are passed through an abrasion device 18 which acts to shear the excess biota from the particles. From abrasion device 18 the carrier particles and the biota sheared therefrom are conducted to a separator 19 from which the carrier particles are fed into the lower section of liquid bath 14 where they float upwardly to rejoin bed 13, the excess biota being discharged through a disposal line 20. This excess growth removal operation is carried out without interrupting the liquid treatment process.

The treated liquid which gathers in bath 14 below the bed is discharged from the reactor through an outlet pipe 21 having a valve 22 therein. The desired liquid level in reactor 10 can be maintained by the setting of valve 22 and/or the size and placement of the discharge of outlet pipe 21.

It may be desirable, depending on the flow rates involved and the reactant concentrations of the liquid being treated, to recycle part of the treated effluent from bath 14 into inlet pipe 11 so that the liquid makes more than one pass through bed 13 and therefore is subjected to more thorough treatment, or to dampen concentration or flow surges in the liquid to be treated.

In practice, in addition to the conventional technique of pre-dissolving gaseous reactants, air or oxygen can be introduced into the reactor in counter current relation to the direction of liquid flow to effect aerobic reactions, and/or the incoming liquid may be sprayed by distributor 12 into a gas head 15 containing oxygen, thereby significantly improving oxygen transfer.

Fluidized-Bed Mode:

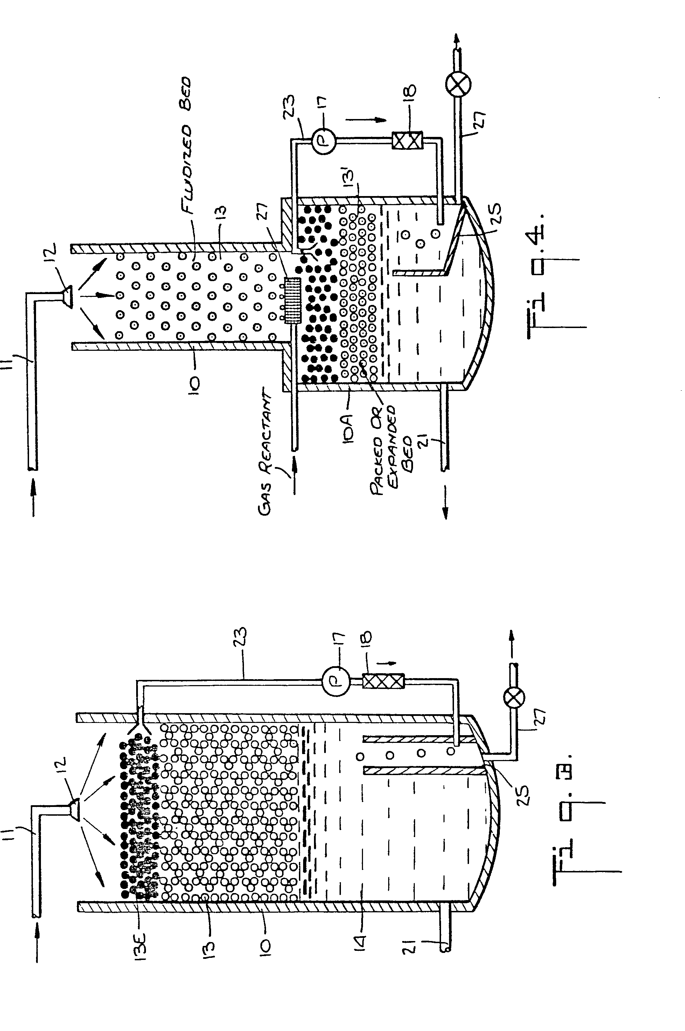

In Fig. 2, the system is shown operating in a fluidized-bed mode, this being effected by causing the incoming liquid to be treated to pass through the media at a velocity sufficient to fluidize the particles in bed 13. The controlling parameters of upflow fluidization are well known. These include particle size, shape and specific gravity as well as liquid specific gravity and velocity. The numerical correlations of Richardson and Zaki and Wen and Yu[*], incorporated herein by reference, define fluidization based on these parameters. These correlations may be modified by one skilled in the art to predict bed expansion and porosity for downwardly fluidized beds. Similarly, the approach taken in the above-identified Scott patent for predicting pressure losses may also be modified for downward fluidization.

Operation of the system in the fluidized-bed mode virtually eliminates the flow distribution and carry-out problems encountered in fluidized-beds of the upflow type, such as the system disclosed in the above-identified Jeris patents; for the downwardly-flowing liquid can easily be dispersed throughout a gas space, and bubble attachment actually tends to keep the media in the system rather than to wash the particles out.

[*]Richardson, J.F.; Zaki, W.N., Trans. Inst. Chem. Eng., 32 35 (195.

[*]Wen, C.Y.; Yu, Y.H., Chem. Eng. Prog. Symp. Ser., 62, 100 (1966).

Another advantageous aspect of the system operating in the fluidized-bed downward flow mode is that excess biological growth can actually be concentrated in the reactor before washing. As the biomass builds up upon the particles, the coated particles tend to migrate toward the downstream zone of the bed. When the expanding fluidized bed reaches a predetermined level, pump 17 is activated to withdraw the media having excess biological growth through pipe 23 having an inlet 24.

The excess growth is pumped through abrasion means 18, from which the mixture of abraded growth and sheared media is fed back to the reactor into a pool zone defined by a clarification chamber 25. With proper adjustment of the flow rate, the sheared media will rise to rejoin the fluidized bed 13, whereas the excess growth will settle in clarification chamber 25 to form a sludge pile 26. The sludge concentrated at the base of chamber 25 is removed therefrom through an outlet 27 having a valve interposed therein.

This sludge concentration technique cannot be carried out in an upflow fluidized bed because the specific gravity of both the carrier particles and the excess growth is greater than that of the liquid; whereas in the downflow fluidized bed in accordance with the invention, the specific gravity of the particles is less than that of the liquid so that it becomes possible to discriminate between the particles and the excess growth. Although this novel sludge control technique has distinct advantages, it is to be understood that conventional fluidized bed growth control techniques may also be used in the present system.

Expanded or
Packed-Bed Mode:

Fig. 3 illustrates the operation of the reactor in the expanded or packed-bed mode. Operation of the system in this mode affords a higher surface area for biological growth than with the synthetic media used in conventional packed bed processes.

A salient advantage of the present invention operated in this mode is that its operation does not have to be interrupted for backwashing as is generally required in conventional packed-bed systems. Media which becomes laden with excess biological growth or entrained-solids may be continuously or intermittently withdrawn from the reactor in a manner whereby excess growth can be sheared from the media and the media returned without interrupting the biological process. That is to say, the pump which sucks excess growth particles from the bed can be operated continuously at a rate which serves to withdraw excess growth particles when the growth build-up is such as to warrant withdrawal, or it can be operated intermittently by sensing the level of the growing bed and actuating the pump when the level exceeds a predetermined value.

In the packed-bed mode of operation, the portion 13E of the media which must be removed will tend to be located toward the upstream end of the reactor, although some particles may migrate toward the downstream end. In the Fig. 3 arrangement, when pump 17 is actuated, pipe 23 draws excess growth particles into abrader 18, the sheared growth and the particles being returned to clarification chamber 25 within the reactor pool.

Combination Mode:

Referring now to Fig. 4, the system illustrated therein operates in a combined fluidized bed and packed or expanded bed mode. To this end, reactor 10 is provided with an expanded lower section 10A. Alternatively, reactor 10 may be of conical configuration to define an expanded lower section.

A potentially significant improvement over currently practiced waste treatment technologies is shown in Fig. 4. The most important limitation currently faced in upflow aerobic fluidized bed is the inability to easily supply $O_2$ to the process. In order to prevent stripping of the biomass from the carrier particles, in most applications the oxygen must be predissolved before entering the reactor. This limits the amount of oxygen which can be supplied to the reactor and requires significant amounts of energy. In addition to this, in most cases pure oxygen rather than air must be used.

Operation of the downflow bioreactor in a fluidized state followed by a packed or expanded state may virtually eliminate oxygen transfer problems and allow air rather than pure oxygen to be used effectively with aerobic fluidized beds. An air diffuser 27 is placed at the bottom of the fluidized section. Since the flow of the liquid is downward, opposing the flow of the air bubbles, oxygen transfer is improved. Any biological growth which may slough off the fluidized bed particles due to the action of the air will be entrained in the lower section resulting in an acceptable effluent quality without clarification.

0025309

Particles with excess growth or solids entrainment will tend to be located at the interface between the fluidized bed and packed or expanded bed zones 13 and 13'. The excess growth is removed as described in prior embodiments.

Although the combination mode described here is the preferred embodiment, it should not be construed to limit the patent to only this combination mode. Other arrangements of fluidized, packed and expanded modes may be used such as having a packed or expanded upper section and a fluidized lower section.

While there has been shown and described a preferred embodiment of a downflow bioreactor in accordance with the invention, it will be appreciated that many changes and modifications may be made therein without, however, departing from the essential spirit thereof.

CLAIMS

1.  A system having relatively low energy requirements for carrying out biologically-catalyzed reactions on an incoming liquid to be treated, said system comprising:

A.  a vertically disposed reactor;

B.  a bed of carrier particles to which biota is attached by seeding the particles with a biological catalyst, said bed being suspended in said reactor and being unconfined therein, said seeded particles having a specific gravity which is less than that of said liquid;

C.  means to supply the incoming liquid to be treated to the upper end of the reactor to cause it to flow downwardly through the particles in the bed, as a result of which a biological growth accumulates on the carrier particles which is adherent thereto and if unchecked renders the system ineffective;

D.  means coupled to said bed for removing excess biological growth material formed on the carrier particles during the operation of the system without detaching said biota therefrom; and

E.  means to withdraw treated liquid from the reactor.

2.  A system as set forth in claim 1, wherein the flow velocity of liquid through the reactor, the diameter of the particles and the specific gravity thereof are such as to render said bed fluidized.

3. A system as set forth in claim 1, wherein the flow velocity of liquid through the reactor, the diameter of the particles and the specific gravity thereof are such as to render said bed expanded or packed.

4. A system as set forth in claim 1, wherein the treated liquid forms a pool below the suspended bed.

5. A system as set forth in claim 1, having a gas space above the bed in the reactor.

6. A system as set forth in claim 5, wherein the upper end of said reactor is enclosing to define said gas space.

7. A system as set forth in claim 5, having means to inject into said gas space an oxygen-containing atmosphere to interact with the incoming liquid fed therein.

8. A system as set forth in claim 1, in which a gaseous reactant is introduced within the reactor, such that the gas bubles rise countercurrent to the flow of said liquid being treated therein.

9. A system as set forth in claim 1, wherein said means for removing excess material from the bed particles include a pump and an abrader, said pump being arranged to suck particles containing excess material from the bed and to pass these particles through said abrader which shears the excess material therefrom, the output of the abrader being fed back into the reactor, the sheared material being carried out of the reactor along with the treated liquid.

10.. A system as set forth in claim 1, wherein said means for removing excess material from the bed particles include a pump, an abrader and a separator, said pump being arranged to suck particles containing excess material from the bed and to pass these particles through said abrader which shears the excess material therefrom, the output of the abrader being fed to said separator which returns the sheared particles to the reactor, the sheared material being discharged from the separator.

11. A system as set forth in claim 10, wherein said pump is coupled to said bed at the upper and lower end zones thereof.

12. A.system as set forth in claim 1, wherein said bed has a clarification chamber formed therebelow and is operated in the fluidized state whereby particles having excess material tend to accumulate in the lower end zone of the bed, and said means to remove excess material from the particles includes a pump and an abrader, said pump being arranged to suck said particles from the lower end zone and to pass them through said abrader whose output is fed into said clarification chamber in which the sheared particles rise to rejoin the bed and the sheared material settles to form a pile at the bottom of the chamber, from which the material is discharged.

13. A system as set forth in claim 1, wherein said bed has a clarification chamber formed therebelow and is operated in the expanded or packed state whereby particles having excess material tend to accumulate in the upper end zone of the bed, and said means to remove excess material from the particles includes a pump and an abrader, said pump being arranged to suck said particles from the upper end zone and to pass them through said abrader whose output is fed into said clarification chamber in which sheared particles rise to rejoin the bed and the sheared material settles to form a pile at the bottom of the chamer from which the material is discharged.

14. A system as set forth in claim 1, wherein said reactor is provided with expanded lower section, one portion of the bed occupying the expanded lower section and operating in the expanded or packed bed mode, another portion of the bed occupying the section above the lower section and operating in the fluidized bed mode, particles with excess material being withdrawn from both sections of the reactor at the interface of the two sections.

15. A system as set forth in claim 4, wherein said means for removing excess material formed on the particles conveys the particles having the excess material removed therefrom to the pool below the suspended bed, whereby the particles float up to rejoin the bed.

Fig.1.

Fig.2.

0025309

Fig. 3.

Fig. 4.

GAS REACTANT

FLUIDIZED BED

PACKED OR
EXPANDED
BED

0025309

0025309

European Patent
Office

**EUROPEAN SEARCH REPORT**

Application number

EP 80 30 2949

| DOCUMENTS CONSIDERED TO BE RELEVANT | | | CLASSIFICATION OF THE APPLICATION (Int. Cl.³) |
|---|---|---|---|
| Category | Citation of document with indication, where appropriate, of relevant passages | Relevant to claim | |
| X | DE - A - 2 905 371 (CHIYODA CHEMI-CAL ENGINEERING) <br><br> * Page 1, claim 1; page 6, paragraph 2; page 7, last two lines; page 8, paragraph 1; page 11, lines 7-29 * <br><br> -- | 1-4,7 | C 02 F 3/12 <br> C 12 M 1/00 <br> B 01 J 8/20 |
| X | US - A - 4 115 266 (KATSUTOSHI OHSHIMA) <br><br> * Column 1, lines 49-54; column 3, lines 1-40 * <br><br> -- | 1,7,8 | **TECHNICAL FIELDS SEARCHED (Int. Cl.³)** |
| P | DE - A - 2 841 011 (METALLGESELL-SCHAFT) <br><br> * Page 1, claim 1 * <br><br> -- | 1 | C 02 F 3/12 <br> 3/06 <br> 3/28 <br> C 12 M 1/00 |
| A | US - A - 3 855 120 (P.W. GARBO) | | |
| A | GB - A - 360 272 (F. SCHIMRIGK) <br><br> ---- | | |

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 27-11-1980 | TEPLY |

EPO Form 1503.1   08.78